# EUROPÄISCHE PATENTSCHRIFT

(11) **EP 2 187 843 B1**
(45) Veröffentlichungstag und Bekanntmachung des Hinweises auf die Patenterteilung: **07.10.2015**
(21) Anmeldenummer: 08785430.3
(22) Anmeldetag: 07.08.2008
(51) Int. Cl.: A61F 2/30, A61L 27/30, A61F 2/44, C04B 41/51, C04B 35/64, B24C 1/00

(54) **ENDOPROTHESENKOMPONENTE**
ENDOPROSTHESIS COMPONENT
ÉLÉMENT ENDOPROTHÉTIQUE

(30) Priorität: 13.09.2007 EP 07018010
(43) Veröffentlichungstag der Anmeldung: 26.05.2010
(73) Patentinhaber: DERU GmbH, 22844 Norderstedt (DE)
(72) Erfinder: LINK, Helmut D., 22397 Hamburg (DE)
(74) Vertreter: Glawe, Delfs, Moll
(86) Internationale Anmeldenummer: PCT/EP2008/006524
(87) Internationale Veröffentlichungsnummer: WO 2009/036845

(56) Entgegenhaltungen:
- EP-A- 0 421 084
- EP-A- 0 633 440
- EP-A- 1 440 669
- EP-A- 1 580 292
- EP-A- 1 674 051
- EP-A- 1 820 622
- DE-A- 3 516 411
- DE-A- 4 322 085
- DE-A- 19 755 536
- DE-U-202005 005 405
- US-A- 5 826 586
- US-A1- 2001 036 530
- US-A1- 2004 029 692
- US-A1- 2004 246 088

## Beschreibung

Die Erfindung betrifft eine Endoprothesenkomponente, die aus einem Keramikwerkstoff geformt ist und bei der der Keramikwerkstoff teilweise mit einer Titan-Legierung beschichtet ist. Ein unbeschichteter oberflächenanteil der Endoprothesenkomponente ist dazu ausgelegt, als Gleitfläche mit einer weiteren Endoprothesenkomponente zusammenzuwirken. Ein beschichteter Oberflächenanteil der Endoprothesenkomponente ist dazu ausgelegt, eine Verbindung mit einem Knochen einzugehen. Die Erfindung betrifft ferner ein Verfahren zur Herstellung einer solchen Endoprothesenkomponente. Die Erfindung betrifft schließlich ein Verfahren zum Herstellen eines Keramikbauteils, auf das die Beschichtung aus der Titan-Legierung aufgebracht werden kann. Bei dem Verfahren wird ein Ausgangsmaterial in Pulverform in eine die Gestalt des Keramikbauteils vorgebenden Form eingebracht. Anschließend wird das Keramikbauteil in mehreren Schritten gesintert.

Keramikwerkstoffe verbinden eine hohe mechanische Festigkeit und Formstabilität mit einer guten biologischen Verträglichkeit. Keramikwerkstoffe kommen deswegen bei der Fertigung von Endoprothesen zum Einsatz.

Bei Endoprothesen, die ein Gelenk ersetzen, muss eine stabile Verbindung zwischen der Endoprothese und dem Knochengerüst hergestellt werden. In dieser Hinsicht sind Keramikwerkstoffe weniger vorteilhaft als andere Materialien, da sie wegen ihrer dichten Oberfläche nur schwer eine Verbindung mit Knochenmaterial eingehen. Aus WO 99/30634 ist es bekannt, den Teil einer Endoprothesenkomponente, der eine Verbindung mit Knochenmaterial eingehen soll, mit einer Titan-Legierung zu beschichten. Die Beschichtung hat eine größere Porosität als der Keramikwerkstoff. Das Knochenmaterial kann in die Poren einwachsen und dadurch eine stabile Verbindung zu der Endoprothesenkomponente herstellen.

Die Titan-Legierung geht mit dem Keramikwerkstoff weder eine chemische noch eine metallurgische Verbindung ein. Die Verbindung zwischen der Titan-Legierung und dem Keramikwerkstoff ist also eine rein mechanische. Gemäß WO 99/30634 soll die Titan-Legierung durch Plasmaspritzen auf den Keramikwerkstoff aufgebracht werden. Für eine gute mechanische Verbindung soll die zu beschichtende Oberfläche des Keramikwerkstoffs rau sein.

Versuche haben gezeigt, dass eine auf diese Weise aufgebrachte Beschichtung nicht mit jeder rauen Oberfläche eine hinreichend stabile Verbindung eingeht. Liegt die Rauigkeit Rₐ unter 2 *µ*m, so hebt sich die aufgebrachte Schicht unter Belastung von dem Keramikmaterial ab.

Der Erfindung liegt die Aufgabe zu Grunde, eine Endoprothesenkomponente der eingangs beschriebenen Art vorzustellen, bei der die Beschichtung besser auf dem Keramikmaterial haftet. Der Erfindung liegt ferner die Aufgabe zu Grunde, ein Verfahren zur Herstellung einer solchen Prothese. Die Aufgaben werden gelöst durch die Merkmale der unabhängigen Ansprüche. Vorteilhafte Ausführungsformen finden sich in den Unteransprüchen. Erfindungsgemäß hat der eine Grenzfläche zu der Beschichtung bildende Teil des Keramikwerkstoffs eine Rauigkeit Rₐ zwischen 2,5 *µ*m und 7 *µ*m, vorzugsweise zwischen 3,5 *µ*m und 5 *µ*m.

Zunächst werden einige Begriffe erläutert. Mit dem Begriff Rauigkeit wird die Unebenheit einer Oberfläche quantitativ angegeben. Die Rauigkeitsangaben im Rahmen der Erfindung beziehen sich auf die mittlere Rauigkeit Rₐ gemäß DIN EN ISO 4288 und 3274.

Die Endoprothesenkomponente ist aus einem Keramikwerkstoff geformt. Die durch den Keramikwerkstoff gebildete Oberfläche ist teilweise mit einer Titan-Legierung beschichtet. Die Titan-Beschichtung bildet nicht zwangsläufig die äußerste Schicht der Endoprothesenkomponente, es kann vielmehr eine weitere Schicht, beispielsweise aus einem Material, das das Knochenwachstum angregt, auf die Titan-Beschichtung aufgebracht sein. Ein Teil der Oberfläche ist unbeschichtet und dazu ausgelegt, als Gleitfläche mit einer weiteren Prothesenkomponente zusammenzuwirken. Der unbeschichtete Bereich und die Gleitfläche sind nicht zwangsläufig identisch.

Im beschichteten Bereich besteht eine Grenzfläche zwischen dem Keramikwerkstoff und der Titan-Legierung. Von der Ausbildung des Keramikwerkstoffs an der Grenzfläche wird die Festigkeit der Verbindung zwischen der Beschichtung und dem Keramikwerkstoff bestimmt. Versuche haben gezeigt, dass die Verbindung zwischen der Beschichtung und dem Keramikwerkstoff unzureichend ist, wenn die Rauigkeit Rₐ des Keramikwerkstoffs an der Grenzfläche kleiner als 2 *µ*m ist. Eine gute Haftung wird ab einer Rauigkeit Rₐ von 2,5 *µ*m erzielt. Die Haftung wird weiter verbessert, wenn die Rauigkeit Rₐ größer als 3,5 *µ*m ist.

Es ist erstrebenswert, dass die Beschichtung in einem einzigen Arbeitsgang aufgetragen werden kann. Wenn die Rauigkeit zu groß ist, müssen mehrere Schichten nacheinander aufgetragen werden, damit die Keramikoberfläche vollständig bedeckt ist und die Schichtdicke überall hinreichend ist. Die Rauigkeit Rₐ des Keramikmaterials an der Grenzfläche sollte deswegen nicht größer als 7 *µ*m, vorzugsweise nicht größer als 5 *µ*m sein.

Die amerikanische Zulassungsbehörde FDA gibt vor, dass die nach ASTM F1147 bestimmte Haftzugfestigkeit zwischen der Beschichtung und dem Keramikwerkstoff mindestens 22 MPa zu betragen hat. Um einen Sicherheitsabstand zu dieser Grenze einzuhalten, sollte die Haftzugfestigkeit zwischen der Beschichtung und dem Keramikwerkstoff bei der erfindungsgemäßen Endoprothesenkomponente bei mindestens 25 Mpa liegen, vorzugsweise bei mindestens 30 MPa, weiter vorzugsweise bei mindestens 40 MPa. Die nach ASTM F1854 bestimmte Schichtdicke der Beschichtung liegt vorzugsweise zwischen 100 *µ*m und 250 *µ*m, vorzugsweise zwischen 150 *µ*m und 200 *µ*m.

Eine gute Verbindung zwischen dem Knochenmaterial und der Beschichtung wird erreicht, wenn die Beschichtung die folgenden Eigenschaften aufweist. Der nach ASTM F1854 bestimmte Porenanteil liegt zwischen 20% und 40%. Die ebenfalls nach ASTM F1854 bestimmte Porenweite beträgt zwischen 30 *µ*m und 70 *µ*m, vorzugsweise zwischen 40 *µ*m und 60 *µ*m.

Als geeignete Keramikwerkstoffe für die erfindungsgemäßen Endoprothesenkomponente haben sich Zirkoniumoxid, Aluminiumoxid sowie Mischungen aus beiden erwiesen. Vom Begriff Titan-Legierung ist reines Titan umfasst.

Die Endoprothesenkomponente ist vorzugsweise eine von mehreren Komponenten einer Bandscheibenprothese. Der als Gleitfläche ausgebildete Teil bildet die Gleitfläche der Bandscheibenprothese.

Das erfindungsgemäße Verfahren zum Herstellen einer Endoprothesenkomponente wird zunächst ein entsprechend geformtes Keramikbauteil ausgewählt, bei dem die zu beschichtende Oberfläche eine Rauigkeit Rₐ zwischen 2,5 *µ*m und 7 *µ*m, vorzugsweise zwischen 3,5 *µ*m und 5 *µ*m aufweist. Auf die zu beschichtende Oberfläche wird eine Titan-Legierung durch Plasmaspritzen aufgebracht. Das Plasmaspritzen ist ein Verfahren, bei dem ein Gas durch einen Lichtbogen geleitet und dadurch ionisiert wird. Das Beschichtungsmaterial wird in Pulverform in das ionisierte Gas eingebracht und von dem Gasstrom auf das zu beschichtende Werkstück transportiert.

Es zeigt sich, dass die Herstellung eines Keramikbauteils, bei dem eine zu beschichtende Oberfläche eine Rauigkeit Rₐ zwischen 2,5 *µ*m und 7 *µ*m aufweist, nicht ganz einfach ist. Bei den üblichen Herstellungsverfahren ist die Oberflächenrauigkeit wesentlich geringer und es ist nicht ohne weiteres möglich, das Keramikbauteil nachträglich mit einer höheren Rauigkeit zu versehen. Zur Herstellung von Keramikbauteilen wird zunächst ein Keramikausgangsmaterial in Form eines Pulvers in eine Form gefüllt wird, die die Gestalt des Keramikbauteils vorgibt. Da sich das Volumen des Keramikbauteils durch nachfolgende Bearbeitungsschritte verringert, ist die Form größer als das fertige Keramikbauteil. Eine stabile innere Struktur des Keramikbauteils wird durch nachfolgendes Sintern erreicht. Durch das Sintern entstehen feste Verbindungen zwischen den Partikeln des Pulvers.

Das Sintern wird in mehreren Schritten durchgeführt. In einem ersten Schritt wird das Keramikbauteil bei einer niedrigeren Temperatur vorgesintert, so dass sich lediglich schmale Brücken zwischen den Körnern des Pulvers herausbilden. Im vorgesinterten Zustand ist das Keramikbauteil zugänglich für eine mechanische Bearbeitung. Einerseits ist die innere Struktur so fest, dass das Keramikbauteil trotz mechanischer Einwirkung seine Form behält. Andererseits lassen sich die Brücken so leicht wieder aufbrechen, dass das Material vergleichsweise leicht bearbeitet werden kann. Je höher die Temperatur des Vorsinterns gewählt wird, desto stabiler sind die Brücken und desto aufwändiger ist die mechanische Bearbeitung. Die Temperatur des Vorsinterns wird deswegen so niedrig wie möglich gewählt, so dass die Brücken gerade eine hinreichende Stabilität haben, um das Keramikbauteil in Form zu halten. Keramikbauteile aus Zirkoniumoxid (ZrO₂) und Aluminiumoxid (Al₂O₃) werden vor der mechanischen Bearbeitung üblicherweise bei Temperaturen von unter 850°C vorgesintert. Typische Schritte der mechanischen Bearbeitung sind Bohren und Fräsen. Es sind keine Schritte der mechanischen Bearbeitung bekannt, mit denen sich die Rauigkeit Rₐ der Oberfläche nachträglich auf Werte zwischen 2,5 *µ*m und 7 *µ*m erhöhen lässt.

Das Keramikbauteil wird bei einer Temperatur zwischen 880°C und 980°C, vorzugsweise zwischen 900°C und 950°C vorgesintert. Anschließend wird die Oberfläche des Keramikbauteils mit einem Strahlgut behandelt. Versuche haben gezeigt, dass bei einem mit dieser Temperatur vorgesinterten Bauteil die Behandlung mit einem Strahlgut zur gewünschten Oberflächenrauigkeit führt. Die innere Struktur des Keramikbauteils nach dem Vorsintern ist also gerade derart, dass mit dem Strahlgut Bruchstücke der gewünschten Größe aus dem Bauteil herausgebrochen werden können.

Bei der erfindungsgemäßen Endoprothesenkomponente wird nur ein Teil der Oberfläche beschichtet, während ein anderer Teil der Oberfläche dazu ausgelegt ist, als Gleitfläche mit einer weiteren Endoprothesenkomponente zusammenzuwirken. Auf der Gleitfläche soll die Rauigkeit der Oberfläche so gering wie möglich sein. Soll eine möglichst glatte Oberfläche erreicht werden, so ist es nicht zweckmäßig, die Rauigkeit der Oberfläche zuvor durch Behandeln mit einem Strahlgut zu erhöhen. Vorzugsweise wird deswegen nur ein Teil der Oberfläche des Keramikbauteils mit dem Strahlgut behandelt, während ein anderer Teil der Oberfläche von der Behandlung mit dem Strahlgut ausgespart bleibt.

Die Teilchengröße des Strahlguts liegt vorzugsweise in derselben Größenordnung wie die Teilchengröße des Keramikausgangsmaterials. Das Strahlgut kann dann besonders wirksam auf das Keramikbauteil einwirken. Allerdings besteht bei der Verwendung eines solchen Strahlguts die Gefahr, dass sich Partikel des Strahlguts in den Lücken des Keramikmaterials festsetzen, die durch die herausgeschlagenen Bruchstücke entstehen. Die Partikel stellen dann Verunreinigungen im Keramikmaterial dar. Verunreinigungen dieser Art können verhindert werden, indem als Strahlgut ein Pulver verwendet wird, das dem Pulver des Keramikausgangsmaterials entspricht. Setzen sich Partikel dieses Materials in dem Keramikbauteil fest, so ändern sie nichts an der homogenen Zusammensetzung des Keramikmaterials.

Die Erfindung wird nachfolgend unter Bezugnahme auf die beigefügten Zeichnung anhand einer vorteilhaften Ausführungsform beispielhaft beschrieben. Es zeigen:
- Fig. 1: eine Bandscheibenprothese in Schnittdarstellung;
- Fig. 2: einen vergrößerten Ausschnitt aus der Bandscheibenprothese gemäß Fig. 1;
- Fig. 3: eine schematische Darstellung der inneren Struktur des Keramikmaterials im Ausgangszustand;
- Fig. 4: die Ansicht aus Fig. 3 nach dem Vorsintern; und
- Fig. 5: die Ansicht aus Fig. 3 nach dem Sintern.

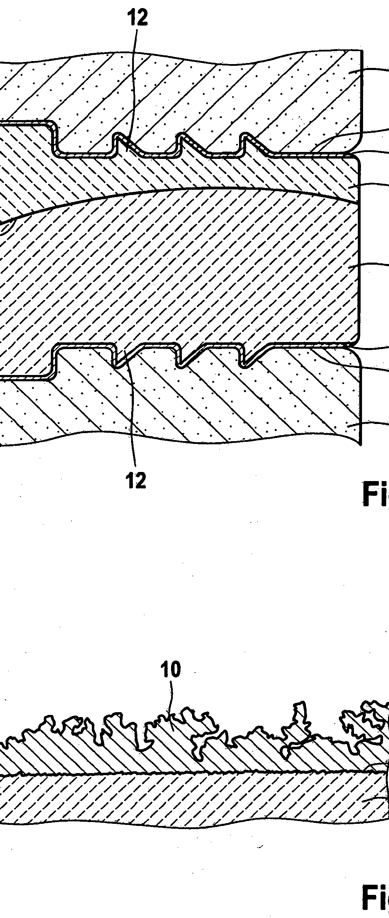

In Fig. 1 ist eine als Bandscheibenprothese ausgebildete Endoprothese in einen Zwischenwirbelraum zwischen zwei Wirbelkörpern 6, 7 eingesetzt. Die Bandscheibenprothese umfasst eine erste Anschlussplatte 1 und eine zweite Anschlussplatte 2. Die erste Anschlussplatte 1 ist eine zur Verbindung mit einem ersten Wirbelkörper 6 ausgebildete Endoprothesenkomponente, die zweite Anschlussplatte 2 ist eine zur Verbindung mit einem zweiten Wirbelkörper 7 ausgebildete Endoprothesenkomponente.

Die erste Anschlussplatte 1 und die zweite Anschlussplatte 2 liegen über zueinander passende Gleitflächen 3 aneinander an. Die Gleitflächen 3 bilden ein Gelenk für Bewegungen zwischen der oberen Anschlussplatte 1 und der unteren Anschlussplatte 2.

In Bereichen 13 der Oberflächen der Anschlussplatten 1, 2, mit denen die Anschlussplatten 1, 2 an dem Knochenmaterial der Wirbelkörper 6, 7 anliegen, sind Vorsprünge 12 ausgebildet. Die vorsprünge 12 haben eine flachere Flanke in der Richtung, in der die Anschlussplatten 1, 2 in den Zwischenwirbelraum eingeschoben werden, und eine steilere Flanke in entgegengesetzter Richtung. Durch die flachere Flanke wird das Einschieben der Anschlussplatten 1, 2 in den zwischenwirbelraum erleichtert. Die steilere Flanke bietet den Anschlussplatten 1, 2 Halt, wenn die Vorsprünge 12 in das Knochenmaterial der Wirbelkörper 6, 7 eingedrungen sind. Die steileren Flanken verhindern, dass die Anschlussplatten 1, 2 in entgegengesetzter Richtung wieder aus dem Zwischenwirbelraum herausgezogen werden können.

Flansche 4, 5 der Anschlussplatten 1, 2 sind dazu bestimmt, an der ventralen Seite der Wirbelkörper 6, 7 anzuliegen. Wegen der Flansche 4, 5 können die Anschlussplatten 1, 2 in dorsaler Richtung nicht über die gewünschte position hinaus in den Zwischenwirbelraum eingeschoben werden.

Die Anschlussplatte 1 und die Anschlussplatte 2 sind aus einem Keramikwerkstoff 9 geformt. Die Gleitflächen 3 an den Anschlussplatten 1, 2 sind an einer Oberfläche des Keramikwerkstoffs 9 ausgebildet. Die Bereiche 13, über die eine Verbindung zu dem Knochenmaterial hergestellt wird, sind mit einer Beschichtung 10 aus einer Titan-Legierung belegt.

Fig. 2 zeigt einen vergrößerten Ausschnitt aus einer Anschlussplatte 1, 2, in dem die Grenzfläche 8 zwischen dem Keramikwerkstoff 9 und der Beschichtung 10 gezeigt ist. Der Keramikwerkstoff 9 hat an der Grenzfläche 8 zur Beschichtung 10 eine Rauigkeit Rₐ, die mindestens 2,5 *µ*m beträgt. Diese Rauigkeit an der Grenzfläche 8 reicht aus, um eine stabile Verbindung mit der Beschichtung 10 einzugehen, für eine stabile Verbindung mit Knochenmaterial reicht sie nicht aus. Die Beschichtung 10 hat eine größere Rauigkeit und größere Porosität als der Keramikwerkstoff 9 und geht deswegen eine stabile Verbindung mit dem Knochenmaterial ein.

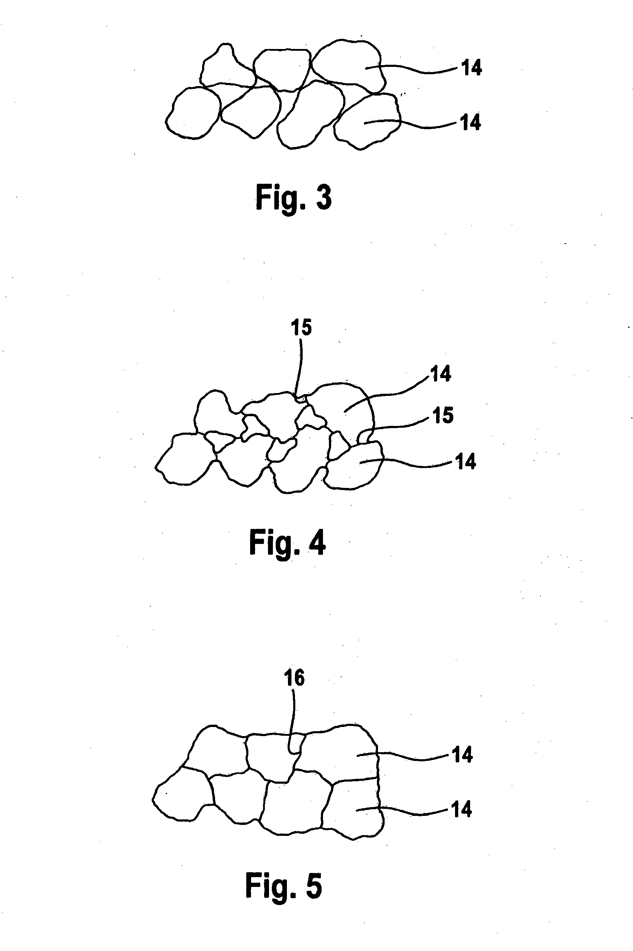

Um ein Keramikbauteil in der Gestalt einer Endoprothesenkomponente herzustellen, bei dem ein Teil der Oberfläche die gewünschte Rauigkeit hat, wird zunächst ein in Pulverform vorliegendes Ausgangsmaterial in eine Form eingebracht, deren Gestalt dem zu erzeugenden Keramikbauteil entspricht. Da das Keramikbauteil durch die nachfolgenden Verfahrensschritte an Volumen verliert, hat die Form größerer Abmessungen als das fertige Keramikbauteil. In der Form wird das Pulver mechanisch verdichtet, beispielsweise durch Schütteln und Druck, die Partikel 14 des Pulvers liegen dann nebeneinander, wie es in Fig. 3 schematisch dargestellt ist. Durch das Vorsintern bei einer Temperatur von 920°C bilden sich in Fig. 4 dargestellte Brücken zwischen den Partikeln 14. Die innere Struktur des Materials ist nun so stabil, dass das Keramikbauteil aus der Form herausgenommen werden kann.

Mit den Brücken 15 hat das Keramikmaterial eine größere Stabilität in seiner Struktur, als man sie für eine mechanische Bearbeitung durch Bohren oder Fräsen wählen würde. Die Stabilität der Struktur ist gerade so eingestellt, dass durch Bestrahlen mit einem Pulver, das dem Ausgangsmaterial entspricht, Bruchstücke aus dem vorgesinterten Keramikmaterial herausgelöst werden können. Die Bruchstücke sind so groß, dass die gewünschte Oberflächenrauigkeit entsteht.

Ist die gewünschte Oberflächenstruktur erreicht und die mechanische Bearbeitung abgeschlossen, wird das Keramikbauteil bei einer Temperatur gesintert, die höher liegt als die Temperatur des Vorsinterns. An den Grenzflächen der bislang nur über die Brücken 15 verbundenen Partikel bilden sich flächige Verbindungen 16. Da die Hohlräume aus dem Inneren verschwinden, nimmt das Volumen des Keramikbauteils weiter ab. Auf dem Teil der Oberfläche, der mit dem Strahlgut behandelt wurde, hat die Oberfläche nun eine Rauigkeit Rₐ von ungefähr 4 *µ*m. Eine auf diesen Oberflächenanteil aufgebrachte Beschichtung aus einer Titan-Legierung haftet fest auf der Oberfläche.

## Patentansprüche

1. Endoprothesenkomponente (1, 2), die aus einem Keramikwertstoff (9) geformt ist und bei der der Keramikwerkstoff (9) teilweise mit einer Titan-Legierung beschichtet ist, wobei ein unbeschichteter Oberflächenanteil dazu ausgelegt ist, als Gleitfläche (3) mit einer weiteren Prothesenkomponente (1, 2) zusammenzuwirken, und wobei ein beschichteter Oberflächenanteil (13) für eine Verbindung mit einem Knochen (6, 7) ausgelegt ist, **dadurch gekennzeichnet, dass** der eine Grenzfläche (8) zu der Beschichtung bildende Teil des Keramikwerkstoffs (9) eine Rauigkeit Rₐ zwischen 2,5 µm und 7 µm, vorzugsweise zwischen 3,5 µm und 5 µm aufweist.

2. Endoprothesenkomponente nach Anspruch 1, **dadurch gekennzeichnet, dass** die Haftzugfestigkeit zwischen der Beschichtung (10) und dem Keramikwerkstoff (9) größer ist als 25 MPa, vorzugsweise größer als 30 MPa, weiter vorzugsweise größer als 40 MPa.

3. Endoprothesenkomponente nach Anspruch 1 oder 2, **dadurch gekennzeichnet, dass** die Oberfläche der Beschichtung (10) eine Rauigkeit Rₐ zwischen 15 µm und 35 µm, vorzugsweise zwischen 20 µm und 30 µm aufweist.

4. Endoprothesenkomponente nach einem der Ansprüche 1 bis 3, **dadurch gekennzeichnet, dass** die Schichtdicke der Beschichtung (10) zwischen 100 µm und 250 µm, vorzugsweise zwischen 150 µm und 200 µm liegt.

5. Endoprothesenkomponente nach einem der Ansprüche 1 bis 4, **dadurch gekennzeichnet, dass** der Porenanteil der Beschichtung (10) zwischen 20% und 40% liegt.

6. Endoprothesenkomponente nach einem der Ansprüche 1 bis 5, **dadurch gekennzeichnet, dass** die Porenweite der Beschichtung (10) zwischen 30 µm und 70 µm, vorzugsweise zwischen 40 µm und 60 µm liegt.

7. Endoprothesenkomponente nach einem der Ansprüche 1 bis 6, **dadurch gekennzeichnet, dass** der Keramikwerkstoff (9) Zirkoniumoxid und/oder Aluminiumoxid umfasst.

8. Endoprothesenkomponente nach einem der Ansprüche 1 bis 7, **dadurch gekennzeichnet, dass** der als Gleitfläche (3) ausgebildete Teil der Keramikoberfläche dazu ausgelegt ist, eine Gleitfläche (3) einer Bandscheibenprothese zu bilden.

9. Verfahren zum Herstellen einer Endoprothesenkomponenten nach einem der Ansprüche 1 bis 8, mit den Schritten:
a. Auswählen eines als Endoprothesenkomponente geformten Keramikbauteils, bei dem eine zu beschichtende Oberfläche eine Rauigkeit Rₐ zwischen 2,5 µm und 7 µm, vorzugsweise zwischen 3,5 µm und 5 µm aufweist.
b. Aufbringen einer Titan-Legierung auf die zu beschichtende Oberfläche durch Plasmaspritzen.

10. Verfahren nach Anspruch 9, **dadurch gekennzeichnet, dass** die Beschichtung in einem Arbeitsgang aufgebracht wird.

## Claims

1. Endoprosthesis component (1, 2) which is formed from a ceramic material (9) and in which the ceramic material (9) is partially coated with a titanium alloy, an uncoated surface portion being designed to interact as slide surface (3) with another prosthesis component (1, 2), and a coated surface portion (13) being designed for connection to a bone (6, 7), **characterized in that** the part of the ceramic material (9) forming an interface (8) to the coating has a roughness Rₐ of between 2.5 µm and 7 µm, preferably of between 3.5 µm and 5 µm.

2. Endoprosthesis component according to Claim 1, **characterized in that** the adhesive pull strength between the coating (10) and the ceramic material (9) is greater than 25 MPa, preferably greater than 30 MPa, more preferably greater than 40 MPa.

3. Endoprosthesis component according to Claim 1 or 2, **characterized in that** the surface of the coating (10) has a roughness Rₐ of between 15 µm and 35 µm, preferably of between 20 µm and 30 µm.

4. Endoprosthesis component according to one of Claims 1 to 3, **characterized in that** the layer thickness of the coating (10) is between 100 µm and 250 µm, preferably between 150 µm and 200 µm.

5. Endoprosthesis component according to one of Claims 1 to 4, **characterized in that** the proportion of the coating (10) taken up by pores is between 20% and 40%.

6. Endoprosthesis component according to one of Claims 1 to 5, **characterized in that** the pore width of the coating (10) is between 30 µm and 70 µm, preferably between 40 µm and 60 µm.

7. Endoprosthesis component according to one of Claims 1 to 6, **characterized in that** the ceramic material (9) comprises zirconium oxide and/or aluminium oxide.

8. Endoprosthesis component according to one of Claims 1 to 7, **characterized in that** the part of the ceramic surface designed as slide surface (3) is intended to form a slide surface (3) of an intervertebral disk prosthesis.

9. Method for producing an endoprosthesis component according to one of Claims 1 to 8, with the following steps:
a. Selecting a ceramic structural part which has been formed as an endoprosthesis component and in which a surface to be coated has a roughness Rₐ of between 2.5 µm and 7 µm, preferably of between 3.5 µm and 5 µm.
b. Applying a titanium alloy, by means of plasma spraying, to the surface to be coated.

10. Method according to Claim 9, **characterized in that** the coating is applied in one operation.

## Revendications

1. Elément endoprothétique (1, 2), qui est formé à partir d'un matériau céramique (9) et dans lequel le matériau céramique (9) est partiellement revêtu d'un alliage de titane, dans lequel une partie de surface non revêtue est destinée à coopérer comme face de glissement (3) avec un autre élément prothétique (1, 2), et dans lequel une partie de surface revêtue (13) est destinée à un assemblage avec un os (6, 7), **caractérisé en ce que** la partie du matériau céramique (9) formant une face limite (8) vers le revêtement présente une rugosité Rₐ comprise entre 2,5 µm et 7 µm, de préférence entre 3,5 µm et 5 µm.

2. Elément endoprothétique selon la revendication 1, **caractérisé en ce que** la résistance à la traction d'éléments adhérents entre le revêtement (10) et le matériau céramique (9) est supérieure à 25 MPa, de préférence supérieure à 30 MPa, de préférence encore supérieure à 40 MPa.

3. Elément endoprothétique selon la revendication 1 ou 2, **caractérisé en ce que** la surface du revêtement (10) présente une rugosité Rₐ comprise entre 15 µm et 35 µm, de préférence entre 20 µm et 30 µm.

4. Elément endoprothétique selon l'une quelconque des revendications 1 à 3, **caractérisé en ce que** l'épaisseur de couche du revêtement (10) se situe entre 100 µm et 250 µm, de préférence entre 150 µm et 200 µm.

5. Elément endoprothétique selon l'une quelconque des revendications 1 à 4, **caractérisé en ce que** la proportion de pores du revêtement (10) se situe entre 20 % et 40 %.

6. Elément endoprothétique selon l'une quelconque des revendications 1 à 5, **caractérisé en ce que** la largeur des pores du revêtement (10) se situe entre 30 µm et 70 µm, de préférence entre 40 µm et 60 µm.

7. Elément endoprothétique selon l'une quelconque des revendications 1 à 6, **caractérisé en ce que** le matériau céramique (9) comprend de l'oxyde de zirconium et/ou de l'oxyde d'aluminium.

8. Elément endoprothétique selon l'une quelconque des revendications 1 à 7, **caractérisé en ce que** la partie de la surface céramique configurée en face de glissement est destinée à former une face de glissement (3) d'une prothèse de disque intervertébral.

9. Procédé de fabrication d'un élément endoprothétique selon l'une quelconque des revendications 1 à 8, comportant les étapes suivantes:
a. sélectionner un composant céramique configuré en élément endoprothétique, dans lequel une surface à revêtir présente une rugosité Rₐ comprise entre 2,5 µm et 7 µm, de préférence entre 3,5 µm et 5 µm.
b. déposer un alliage de titane sur la surface à revêtir par projection au plasma.

10. Procédé selon la revendication 9, **caractérisé en ce que** le revêtement est déposé en une seule opération.
